Europäisches Patentamt

**European Patent Office**

Office européen des brevets

(19)

(11)   **EP 1 036 054 B1**

(12)   **EUROPÄISCHE PATENTSCHRIFT**

(45) Veröffentlichungstag und Bekanntmachung des Hinweises auf die Patenterteilung:
   **17.10.2001   Patentblatt 2001/42**

(51) Int Cl.$^7$: **C07C 17/156**, C07C 19/045

(21) Anmeldenummer: **98965167.4**

(22) Anmeldetag: **19.11.1998**

(86) Internationale Anmeldenummer:
   **PCT/EP98/07444**

(87) Internationale Veröffentlichungsnummer:
   **WO 99/28280 (10.06.1999 Gazette 1999/23)**

(54) **VERFAHREN ZUR HERSTELLUNG VON 1,2-DICHLORETHAN DURCH OXICHLORIERUNG**

   METHOD FOR PRODUCING 1,2-DICHLOROETHANE BY OXYCHLORINATION

   PROCEDE POUR PRODUIRE DU 1,2-DICHLOROETHANE PAR OXYCHLORATION

(84) Benannte Vertragsstaaten:
   **BE DE ES FR GB IT NL**

(30) Priorität: **01.12.1997   DE 19753165**

(43) Veröffentlichungstag der Anmeldung:
   **20.09.2000   Patentblatt 2000/38**

(73) Patentinhaber: **Vinnolit Monomer GmbH & Co. KG**
   **85737 Ismaning (DE)**

(72) Erfinder:
   • **ERTL, Horst**
     **D-84524 Neuoetting (DE)**
   • **KAMMERHOFER, Peter**
     **D-84508 Burgkirchen (DE)**

   • **SCHWARZMAIER, Peter**
     **D-84556 Kastl (DE)**
   • **MIELKE, Ingolf**
     **D-84508 Burgkirchen (DE)**

(74) Vertreter: **Boeters, Hans Dietrich, Dr. et al**
   **Patentanwälte Boeters & Bauer,**
   **Bereiteranger 15**
   **81541 München (DE)**

(56) Entgegenhaltungen:
   **EP-A- 0 103 940        DE-A- 1 468 488**
   **DE-A- 19 546 068       GB-A- 1 256 245**
   **GB-A- 1 351 700**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

**Beschreibung**

Unter "Oxichlorierung" versteht man die Umsetzung eines Alkens - hier Ethen - mit Chlorwasserstoff und Sauerstoff oder einem Sauerstoff enthaltenden Gas wie Luft, unter Bildung eines gesättigten chlorierten Alkans - hier 1,2-Dichlorethan, im folgenden "EDC" genannt. Die Reaktion erfolgt nach der Gleichung

$$C_2H_4 + 2HCl + \tfrac{1}{2}O_2 \Leftrightarrow Cl\text{-}CH_2\text{-}CH_2\text{-}Cl + H_2O.$$

Das Reaktionsnebenprodukt Wasser kann also mit dem nichtumgesetzten Ausgangsmaterial Chlorwasserstoff die korrosive Salzsäure bilden, so daß entsprechend resistente - und damit teure - Materialien für die Apparaturen eingesetzt werden müssen.

In einer häufig im großindustriellen Ausmaß ausgeübten Ausführungsform dieses Verfahrens dient als Katalysator ein Wirbelbett, wobei der Katalysator im wesentlichen aus Kupferchlorid auf einem Aluminiumoxidträger besteht.

Bei den üblichen industriellen Verfahren wird der aufgewirbelte Katalysator im Oberteil des Oxichlorierungsreaktors durch mehrere hintereinandergeschaltete Cyclone abgeschieden und so zum größten Teil im Reaktor zurückgehalten. Allerdings geht hierbei ein kleiner Anteil mit dem Reaktionsabgas über und gelangt so in die EDC-Aufarbeitung, wo er abgetrennt werden muß.

Aus der DE-A-41 32 030 ist ein Verfahren zur Entfernung des Katalysatorabriebs bekannt, der bei der Herstellung von EDC nach dem Oxichlorierungsverfahren in der Reaktionszone anfällt und aus der Reaktionszone mit dem rohen EDC-Gasstrom abgeführt wird, das dadurch gekennzeichnet ist, daß man den abgeführten Katalysatorabrieb in einer trocken betriebenen Reinigungszone aus dem rohen EDC-Gasstrom abtrennt. Bevorzugte Ausführungsformen dieses Verfahrens sind dadurch gekennzeichnet, daß man den Katalysatorabrieb an einem Staubabscheider oder in einem Elektrofilter als Reinigungszone abtrennt, daß der Staubabscheider mit Schlauchfiltern ausgerüstet ist, die man mit komprimiertem Kreislaufgas reinigt, daß man den in der Reinigungszone abgeschiedenen Katalysatorabrieb in einer nachgeschalteten Desorptionszone von adsorbierten Reaktionsprodukten befreit, daß man die Desorptionszone bei einer Temperatur von 50 bis 350 °C, insbesondere 150 bis 180 °C, durch Begasen oder im Unterdruck betreibt, daß man zur Begasung Luft, Stickstoff oder Kreislaufgas verwendet und daß man den Katalysatorabrieb 0,5 bis 5 Stunden, vorzugsweise 1 bis 2 Stunden, lang bei erhöhter Temperatur in der Desorptionszone behandelt. Mit diesem Verfahren wird vermieden, daß bei der Ausschleusung des gebildeten Wassers sowie des bei der Aufarbeitung eingesetzten Waschwassers ein mit Schwermetall und anorganischem Schlamm verunreinigtes Abwasser entsteht. Der abgetrennt Katalysatorfeinanteil muß jedoch verworfen und sachgemäß entsorgt werden.

Die DE-A-195 46 068 betrifft ein Verfahren zur Reduzierung des Katalysatorverbrauchs und verunreinigter Katalysatorabfälle bei der Herstellung von EDC nach dem Oxichlorierungsverfahren an einem kupferhaltigen Wirbelbettkatalysator in einer Reaktionszone, bei dem man den Katalysatorabrieb in einer trockenbetriebenen Trennzone aus dem rohen EDC-Gasstrom abtrennt, das dadurch gekennzeichnet ist, daß man den Katalysatorabrieb klassiert und bestimmte Kornfraktionen wieder in die Reaktionszone zurückführt. Bevorzugte Ausgestaltungen dieses Verfahrens bestehen darin, daß man den Katalysatorabrieb in eine Grob- und eine Feinfraktion klassiert, daß die Grobfraktion einer Korngröße > 5 µm und die Feinfraktion einer Korngröße < 5 µm entspricht, daß man die Grobfraktion in die Reaktionszone zurückführt, daß man die Feinfraktion bei 300 bis 800 °C, vorzugsweise 600 bis 800 °C, thermisch nachbehandelt, daß man die nachbehandelten Abgase in einen Verbrennungsofen einleitet, daß der Verbrennungsofen eine Temperatur oberhalb 900 °C, vorzugsweise mehr als 1000 °C, aufweist, daß man aus der Feinfraktion Kupfer und/oder Aluminium zurückgewinnt, daß man die Feinfraktion kontrolliert deponiert und daß die Doxine und/oder Furane aus dem Katalysatorabrieb entfernt werden. Mit diesem Verfahren werden also die Nachteile des Verfahrens gemäß DE-A-41 32 030 überwunden, allerdings um den Preis eines erheblichen Aufwands an Apparaten und Arbeit bei deren Bedienung und Wartung.

Beiden bekannten Verfahren ist gemeinsam, daß die Abtrennung der ausgetragenen Katalysatorfeinanteile in einer vom Reaktor getrennten Zone erfolgt.

Es wurde nun gefunden, daß die Austragung des Katalysators aus dem Reaktor selbst vermieden werden kann, wenn man den Katalysator im Oberteil des Reaktors durch eine Feinstfiltration praktisch vollständig zurückhält.

Die Erfindung betrifft somit ein Verfahren zur Herstellung von EDC durch Oxichlorierung, wobei an einem Wirbelbett aus einem kupferhaltigen Katalysator Ethen mit Chlorwasserstoff und Sauerstoff oder einem Sauerstoff enthaltenden Gas in Reaktion treten und wobei das aus dem Reaktor austretende Reaktionsgas im Reaktor durch eine Feinstfiltration von Katalysator befreit und dieser so im Reaktor zurückgehalten wird.

Unter "Feinstfiltration" wird ein Vorgang verstanden, der die Zurückhaltung des Oxichlorierungskatalysator-Feinanteils bewirkt. Während die bisher üblichen Cyclone einen Feinanteil unter 10 µm in den Produktstrom gelangen ließen, werden erfindungsgemäß Teilchen bis zu 1 µm zurückgehalten, das heißt praktisch der gesamte Katalysator.

Überraschenderweise wurde weiterhin gefunden, daß erfindungsgemäß auf die Abtrennung des Katalysatorgrobanteils mittels der Cyclone verzichtet werden kann. Es werden somit der Grob- und Feinanteil des Katalysators in einem Schritt durch die Feinstfiltration zurückgehalten. Dies bringt eine Reihe von Vorteilen mit sich:

Durch den Verzicht auf die Cyclone wird nicht nur der apparative Aufwand und die umständliche Wartung dieser schlecht zugänglichen Bauteile vermieden, sondern man kann auch die Bauhöhe der Reaktoren signifikant verringern. Damit verbilligt sich der Reaktor erheblich und natürlich sinkt auch die Raumbeanspruchung in der Anlage und damit der bauliche Aufwand.

Demgegenüber sind die zur Feinstfiltration erforderlichen - an sich bekannten - Einrichtungen leicht in gut zugänglicher Form im Oberteil des Reaktors zu installieren, beispielsweise jeweils in eigenen Stutzen, die eine einfache Wartung oder einen raschen Austausch des Filterapparates mit nur kurzen Betriebsunterbrechungen erlauben, weiterhin erlaubt es eine solche Konstruktion, während des laufenden Betriebs einzelne Apparate außer Betrieb zu setzen.

Geeignet sind beispielsweise Filterkerzen-Apparate aus für die EDC-Herstellung geeigneten Materialien, beispielsweise Metallen, Legierungen, Glas oder Keramik, vorzugsweise mit Filterkerzen aus porösem, hinreichend korrosionsresistentem Metall wie gesinterten Metallpulvern oder Drahtgeweben oder -vliesen aus Edelstahl oder hochkorrosionsfesten Legierungen, wie sie unter den Bezeichnungen ® INCONEL (Marke der Firma Inco Ltd.; Nickel-Chrom-Legierung), ® MONEL (Marke der Firma Inco Ltd.; Nickel-Kupfer-Legierung), ® HASTELLOY (Nickel-Legierung) handelsüblich sind, sowie aus porösen keramischen Materialien.

Brauchbar sind weiterhin Gewebefilter aus hinreichend temperaturresistenten, insbesondere fluorierten Kunststoffen wie Polytetrafluorethylen, beispielsweise Schlauchfilter oder Patronen.

Geeignet sind alle Feinstfilter, die Partikel von 1 µm und darüber zurückhalten, also vorzugsweise nur Partikel unter 0,8 µm, insbesondere unter 0,5 µm oder sogar unter 0,2 µm passieren lassen.

Die Abscheidung der an den Filtermaterialien aufgebauten Filterkuchen aus Katalysator erfolgt - wie üblich - vorteilhaft durch Durchleiten von Gas im Gegenstrom, vorzugsweise Reaktionsgase (Edukte), Inertgase oder im Kreislauf geführtem Gas (Kreisgas), zum Beispiel in Pulsen, vorzugsweise im regelmäßigen zeitlichen Abstand, oder sobald sich eine vorherbestimmte Dicke des Filterkuchens aufgebaut hat und/oder sich ein entsprechender Druckabfall eingestellt hat.

Es ist sehr überraschend, daß mit dieser einfachen Anordnung des Feinstfilters im Reaktor selbst ein Austrag des Katalysators zuverlässig vermieden werden kann und so der bisher erforderliche Aufwand mit der Rückgewinnung und Aufarbeitung, die stets mit Verlusten verbunden war, entfallen kann. Darüber hinaus wird auch die durch den Katalysator verursachte Abrasion in den Anlagenteilen bis zur Austragung des Feinanteils vermieden.

Durch die Aufwirbelung des Katalysators wird im Laufe der Zeit das Korngrößenspektrum zu kleineren Teilchen verschoben.

Da dieser Vorgang mit einer Vergrößerung der wirksamen Oberfläche verbunden ist, ist dies mit einer Erhöhung der Katalysatoraktivität verknüpft. Sofern dies unerwünscht ist oder nach längerem Betrieb es erforderlich wird, kann der unerwünschte Feinanteil bei einer kurzzeitigen Unterbrechung des Verfahrens leicht separiert werden. Die bei den bekannten Verfahren auftretenden Nachteile mit dem kontinuierlichen Austrag des Feinanteils treten somit hier nicht auf.

Es wurde schon ein Verfahren zur Aufarbeitung von 1,2-Dichlorethan aus der Oxichlorierung vorgeschlagen, das dadurch gekennzeichnet ist, daß die gasförmigen Produkte aus dem Oxichlorierungsreaktor von mitgerissenem Katalysator befreit und anschließend saure Anteile in einer Waschzone mit einer alkalischen Waschlösung ausgewaschen werden (deutsche Patentanmeldung 197 03 857.3 vom 03.02.1997). Bevorzugte Ausgestaltungen dieses Verfahrens sind dadurch gekennzeichnet, daß die alkalische Waschlösung im Gegenstrom geführt wird und daß zwischen Katalysatorentfernung und Waschzone ein Teilstrom der gasförmigen Produkte abgezweigt und analytisch untersucht wird. Vorgeschlagen wurde weiterhin eine Vorrichtung zur Durchführung des genannten Verfahrens, die gekennzeichnet ist durch einen Oxichlorierungsreaktor, eine Gasabführungsleitung, die über einen Katalysatorabscheider in eine Waschzone geführt wird, in der saure Bestandteile mit einer alkalischen Waschlösung abgetrennt werden. Vorteilhaft wird weiterhin eine Abzweigleitung zwischen Katalysatorabscheider und Waschzone für die analytische Überwachung der Reaktion vorgesehen.

Es wurde weiterhin ein Verfahren zur Herstellung von 1,2-Dichlorethan aus Ethylen, Chlorwasserstoff und Sauerstoff beziehungsweise einem sauerstoffhaltigen Gas, wobei man nichtumgesetzten Chlorwasserstoff aus dem Reaktionsgemisch mit Wasser auswäscht, im Waschwasser einen Parameter bestimmt und diesen zur zumindest teilweisen Neutralisation des Chlorwasserstoffs heranzieht, vorgeschlagen, das dadurch gekennzeichnet ist, daß man den Parameter zusätzlich zur Steuerung der eingesetzten Chlorwasserstoffmenge verwendet. Vorteilhaft wird hierbei als Parameter die elektrische Leitfähigkeit bestimmt, das Waschwasser im Kreislauf geführt und/oder die Leitfähigkeit im ablaufenden und zusätzlich im zulaufenden Waschwasser gemessen (deutsche Patentanmeldung 196 31 382.1 vom 02.08.1996). Auch dieses Verfahren läßt sich vorteilhaft mit dem erfindungsgemäßen Verfahren kombinieren, gegebenenfalls auch in Kombination mit dem in der deutschen Patentanmeldung 197 03 857.3 vorgeschlagenen Verfahren.

Im übrigen wird das Oxichlorierungsverfahren in an sich bekannter Weise durchgeführt:

Die Temperaturen in der Reaktionszone des Reaktors betragen 200 bis 270 °C, vorzugsweise 215 bis 230 °C und insbesondere 220 bis 225 °C. Die Drükke liegen bei $2,5 \cdot 10^5$ bis $5 \cdot 10^5$ Pa, vorzugsweise $3 \cdot 10^5$ bis $4 \cdot 10^5$ Pa, insbesondere bei $3,4 \cdot 10^5$ bis $3,5 \cdot 10^5$ Pa (jeweils Überdruck).

Pro Mol Ethen werden bis 1,92 mol Chlorwasserstoff eingesetzt sowie bis 0,53 mol Sauerstoff, wobei - in an sich bekannter Weise - darauf geachtet wird, daß das Ethen beziehungsweise der Sauerstoff erst mit dem Katalysator in Kontakt tritt, bevor er mit dem anderen Reaktionspartner zusammenkommt (zum Beispiel WO-A-96/26003) beziehungsweise sonst, in bekannter Weise, so verfahren wird, daß explosive Gasgemische vermieden werden.

Auch die Aufarbeitung des Reaktionsgases erfolgt in üblicher Weise. Hierzu kann beispielsweise auf die eingangs genannten Druckschriften verwiesen werden.

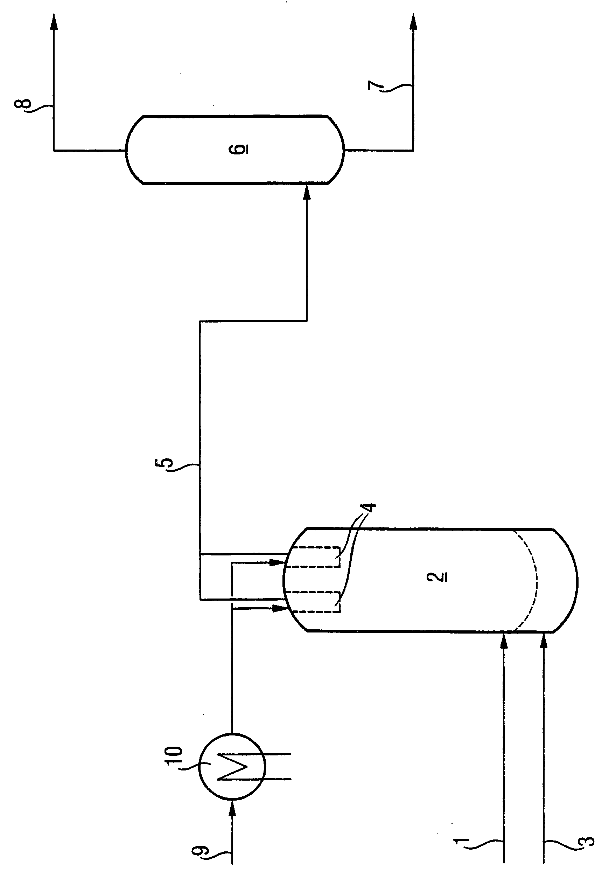

In dem folgenden Beispiel wird die Erfindung anhand der Figur noch näher erläutert.

Beispiel

5910 Nm$^3$/h Chlorwasserstoff mit einer Temperatur von 150 °C und 1600 Nm$^3$/h Sauerstoff, erhitzt auf 110 °C, werden über die Leitung 1 gemeinsam in den Reaktor 2 eingeleitet.

3000 Nm$^3$/h Ethylen werden gemeinsam mit dem Kreisgas auf 150 °C erhitzt und über die Leitung 3 dem Reaktor 2 zugeführt. Im Reaktor 2 befinden sich 40 t Wirbelbett-Katalysator (Aluminiumoxid mit einem Kupfergehalt von 4 Gew.-%) mit folgender Kornverteilung:

| Korngröße [μm] | Anteil (Durchgang) [Gew.-%] |
|---|---|
| < 20 | 4 |
| < 32 | 6 |
| < 41 | 26 |
| < 50 | 54 |
| < 61 | 82 |
| < 82 | 96 |

Die Reaktionswärme wird über einen Heißwasserkreislauf unter Dampfgewinnung abgeführt. Das Reaktionsgas durchströmt nach Verlassen des Wirbelbetts zur Abscheidung von mitgerissenen Katalysatorteilchen im oberen Teil des Reaktors den Feinstfilter 4, in dem der Katalysator praktisch vollständig abgeschieden wird. Das vom Katalysator befreite Reaktionsgas der Temperatur von 210 °C wird über die Leitung 5 in die Quenchkolonne 6 geleitet, wo das Produktionswasser kondensiert und über die Leitung 7 der Abwasseraufarbeitung zugeführt wird. Der Kupfergehalt im Quenchwasser beträgt < 0,05 mg/l. Der Kopfstrom, bestehend im wesentlichen aus EDC und Kreisgas, wird über die Leitung 8 der EDC-Aufarbeitung zugeführt.

Der Feinstfilter 4 wird differenzdruckgesteuert über die Leitung 9 mit Stickstoff, der im Vorheizer 10 auf 180 °C erhitzt wird, abgereinigt. Die Rückhalterate ist > 99,99 %.

## Patentansprüche

**1.** Verfahren zur Herstellung von 1,2-Dichlorethan durch Umsetzung von Ethen mit Chlorwasserstoff und Sauerstoff oder einem sauerstoffhaltigen Gas an einem kupferhaltigen Wirbelbettkatalysator, **dadurch gekennzeichnet, daß** innerhalb des Reaktor durch Filtration alle Katalysatorteilchen > 1 μm zurückgehalten werden.

**2.** Verfahren nach Anspruch 1, **dadurch gekennzeichnet, daß** die Filtration mittels Filterkerzen, Schlauchfiltern oder Patronenfiltern erfolgt.

**3.** Verfahren nach Anspruch 2, **dadurch gekennzeichnet, daß** Filterkerzen aus gesintertem Metall oder aus Keramik eingesetzt werden.

## Claims

**1.** A process for the production of 1,2-dichloroethane comprising a reaction of ethane with hydrogen chloride and oxygen or a gas containing oxygen on a fluidised bed catalyst containing copper, such process being **characterised by** the retention in the reactor of all particles of the catalyst in excess of 1μm as a result of filtration.

**2.** The process of claim 1 wherein the filtration is achieved by the use of filter candles, tube filters or cartridge filters.

**3.** The process of claim 2 wherein the filter candles are made of sintered metal or a ceramic material.

## Revendications

**1.** Procédé de préparation du 1,2-dichloroéthane par réaction de l'éthène avec le chlorure d'hydrogène et l'oxygène ou un gaz contenant de l'oxygène sur un catalyseur à lit fluidisé contenant du cuivre, **caractérisé en ce que** l'on retient à l'intérieur du réacteur toutes les particules du catalyseur > 1 μm par filtration.

**2.** Procédé selon la revendication 1, **caractérisé en ce que** la filtration a lieu au moyen de bougies de filtre, de filtres à manches ou de filtres à cartouches.

**3.** Procédé selon la revendication 2, **caractérisé en**

**ce que** des bougies de filtre à base de métal fritté ou de céramique sont mises en oeuvre.